# EUROPÄISCHE PATENTANMELDUNG

(11) **EP 3 693 751 A1**
(43) Veröffentlichungstag der Anmeldung: **12.08.2020**
(21) Anmeldenummer: 19156482.2
(22) Anmeldetag: 11.02.2019
(51) Int. Cl.: G01R 33/54, G01R 33/56, A61B 5/055, G01R 33/565

(54) **AUTOMATISCHE AUSWAHL EINER OPTION FÜR DIE UNTERDRÜCKUNG EINER GEWEBEKOMPONENTE FÜR MAGNETRESONANZBILDGEBUNG**

(71) Anmelder: Siemens Healthcare GmbH, 91052 Erlangen (DE)
(72) Erfinder: Grodzki, David, 91058 Erlangen (DE)

(57) **Zusammenfassung**

Die Erfindung betrifft ein Verfahren zum automatischen Steuern eines Untersuchungsablaufs in einer MR-Anlage (9) bei der Aufnahme von MR-Signalen in einem Untersuchungsabschnitt einer Untersuchungsperson (13), der zwei Gewebekomponenten mit zwei unterschiedlichen MR-Resonanzfrequenzen aufweist, mit den Schritten:
- Bestimmen eines Untersuchungsablauf für die Untersuchung des Untersuchungsabschnitts,
- Bestimmen, ob der Untersuchungsablauf eine Bildgebungssequenz aufweist, bei der eine der beiden Gewebekomponenten unterdrückt werden soll und für die zumindest zwei verschiedene Unterdrückungsoptionen existieren, diese eine Gewebekomponente bei der MR-Signalaufnahme zu reduzieren, wobei wenn dies der Fall ist:
- Bestimmen eines Ablaufparameters der Untersuchung,
- Auswählen eine der beiden Unterdrückungsoptionen in Abhängigkeit von dem bestimmten Ablaufparameter für die Bildgebungssequenz.

## Beschreibung

Die vorliegende Erfindung betrifft ein Verfahren zum automatischen Steuern eines Untersuchungsablaufs in einer MR-Anlage in einem Untersuchungsabschnitt der zwei Gewebekomponenten mit zwei unterschiedlichen MR-Resonanzfrequenzen aufweist. Weiterhin wird die zugehörige MR-Anlage bereitgestellt, ein Computerprogramm mit Programmmittel und ein elektronisch lesbarer Datenträger.

Ein großer Vorteil der MRT (Magnetresonanztomographie) im Vergleich zu röntgenbasierten Bildgebungsverfahren wie beispielsweise die Computertomografie, CT, ist der hohe Weichteilkontrast, der mit der MRT möglich ist. Je nach Fragestellungen können verschiedene Gewichtungen vorgenommen werden, die eine optimale Darstellung des Gewebes ermöglichen. Der menschliche Körper weist Bereiche auf, in denen Fettsignalanteile und Wassersignalanteile im MR-Signal vorhanden sind. Fett und Wasser haben eine unterschiedliche Resonanzfrequenz. Für einige medizinische Fragestellungen ist eine Unterdrückung des Fettsignals wünschenswert. Meist wird hierzu ein spektral selektiver und/oder ein T1 selektiver HF-Vorpuls verwendet oder die DIXON-Methode verwendet, die darauf beruht, dass Fett und Wasser bei bestimmten Echozeiten verschiedene Phasenlagen haben.

Je nach Anwendung haben diese beiden Ansätze unterschiedliche Vorteile oder Nachteile, und die untersuchenden Radiologen haben oft unterschiedliche Präferenzen. Jede der Verfahren haben Vor- und Nachteile, wobei nicht jedes der beiden Verfahren bei jeder Situation anwendbar ist.

Neuere MRT-Anlagen zielen darauf ab, diese für Kundengruppen zugänglich zu machen, die bisher keine MRT-Geräte verwendet haben. Für diese Kundengruppen ist es oft schwer, gut geschultes MR-Bedienpersonal zu finden. Wie oben erwähnt, bestehen viele Einstellungsmöglichkeiten, um verschiedene Kontraste zu erzeugen. Hierfür ist gut geschultes Bedienpersonal notwendig. Das Bedienpersonal hat Interesse an einer einfach ausgeführten Benutzerschnittstelle, an weiteren Automatisierungen wie beispielsweise die automatische Wahl einer Schichtposition zur Erzeugung der MR-Bilder oder die Wahl des Gesichtsfeldes, Field of View, FOV.

Wie oben erwähnt ist für die Wahl eines geeigneten Fettunterdrückungsmodus ein gewisses Expertenwissen notwendig, da die beste Wahl von verschiedenen Faktoren abhängt.

Es ist daher eine Aufgabe der vorliegenden Erfindung die Wahl der Unterdrückung einer Gewebekomponente, die im MR-Signal unerwünscht ist, weiter zu vereinfachen.

Diese Aufgabe wird durch die Merkmale der unabhängigen Ansprüche gelöst. In den abhängigen Ansprüchen sind weitere Ausführungsformen beschrieben.

Erfindungsgemäß wird ein Verfahren zum automatischen Steuern eines Untersuchungsablaufs in einer MR-Anlage bereitgestellt bei der Aufnahme von MR-Signalen in einem Untersuchungsabschnitt einer Untersuchungsperson. Der Untersuchungsabschnitt weist zwei Gewebekomponenten mit zwei unterschiedlichen MR-Resonanzfrequenzen auf. Bei diesem automatischen Verfahren wird ein Untersuchungsablauf für die Untersuchung des Untersuchungsabschnitts bestimmt. Weiterhin wird festgestellt, ob der Untersuchungsablauf eine Bildgebungssequenz aufweist, bei der eine der beiden Gewebekomponenten unterdrückt werden soll und für die zumindest zwei verschiedene Unterdrückungsoptionen existieren, diese eine Gewebekomponente bei der MR-Signalaufnahme zu reduzieren. Wenn dies der Fall ist wird ein Ablaufparameter der Untersuchung bestimmt und automatisch eine der beiden Unterdrückungsoptionen in Abhängigkeit von dem bestimmten Ablaufparameter für die Bildgebungssequenz ausgewählt.

Durch die Bestimmung des Ablaufparameters ist eine Größe vorhanden, die dazu verwendet wird, eine der zumindest zwei Unterdrückungsoptionen auszuwählen. Der Benutzer ist somit von dieser Aufgabe befreit und das System kann automatisch die beste Unterdrückungsoption auswählen für die vorliegende Situation und die vorliegende Untersuchung. Das kann bedeuten, dass sich auch die dazugehörigen Bildgebungssequenzen unterscheiden, da beide Optionen unterschiedliche Effekte ausnützen und daher verschiedene Abfolgen von Gradienten und HF-Pulsen notwendig sein können.

Die zumindest zwei verschiedenen Unterdrückungsoptionen weisen eine erste Unterdrückungsoption auf, bei der die beiden Gewebekomponenten mit einem spektral selektiven HF-Puls unterschiedlich angeregt werden, wobei die zweite Unterdrückungsoption die unterschiedliche Phasenlage der beiden Gewebekomponenten bei einer oder mehreren Echozeiten verwendet.

Bei der Bestimmung des Ablaufparameters kann beispielsweise ein Inhomogenitätsparameter mit der MR-Anlage bestimmt werden. Dieser Inhomogenitätsparameter wird dann verwendet, um automatisch eine der beiden Unterdrückungsoptionen automatisch auszuwählen.

Der Inhomogenitätsparameter kann eine Homogenität des Polarisationsfeldes B₀, eine Homogenität des B₁-Feldes zur Einstrahlung der HF-Pulse während der Bildgebungssequenz beinhalten und/oder eine Größe des induzierten Wirbelstroms, die sogenannten eddy currents.

Wenn beispielsweise der Inhomogenitätsparameter größer als ein definierter Grenzwert ist, so kann das System automatisch die zweite Unterdrückungsoption, die auf dem DIXON-Verfahren beruht, verwenden. Das DIXON-Verfahren hat den Vorteil, dass es robuster ist gegenüber Inhomogenitäten, beispielsweise des B₀-Feldes, bei Wirbelströmen oder des B₁-Feldes. Bei größeren Inhomogenitäten ist es schwierig, die nahe beieinanderliegenden spektralen Komponenten so zu trennen, dass die eine angeregt wird und die andere nicht, wie es bei der ersten Option notwendig ist.

Ein weiterer Ablaufparameter, der bestimmt werden kann, ist der Abstand zu einem Zentrum der MR-Anlage, in dem der Untersuchungsabschnitt bei der Aufnahme der MR-Signale positioniert ist. Dieser Abstand kann ebenfalls berücksichtigt werden bei der automatischen Auswahl von einer der beiden Unterdrückungsoptionen.

Ist beispielsweise der Abstand zum Zentrum größer als ein Grenzwert, so kann bevorzugt die zweite Unterdrückungsoption, die auf der DIXON-Methode beruht verwendet werden. Bei Messungen die außerhalb des Zentrums der MR-Anlage durchgeführt werden ist die Homogenität der benötigten Felder oft nicht so gut, dass eine spektrale Sättigung zufriedenstellend möglich ist. Daher ist es hier vorteilhaft, die Unterdrückungsoption zu wählen, bei der die unterschiedliche Phasenlage der beiden Gewebekomponenten verwendet wird zur Trennung der beiden Signalkomponenten.

Die erste Unterdrückungsoption mit der Verwendung eines spektral selektiven HF-Pulses kann verwendet werden, wenn der Inhomogenitätsparameter kleiner als ein Grenzwert ist und wenn der Abstand vom Zentrum kleiner als ein weiterer Grenzwert ist. Der Grenzwert für den Inhomogenitätsparameter kann bei 20, 25 oder 30 % des spektralen Unterschieds zwischen den beiden Komponenten liegen, bei Fett und Wasser also bei beispielsweise 0,25 * 3,5 ppm. Der Grenzwert für den Abstand zum Zentrum kann, gemessen am größtmöglichen Gesichtsfeld, FOVmax, bei 30, 40, 50 oder 60 % von FOVmax liegen.

Wenn hingegen bei einem der beiden Parameter, der Abstand zum Zentrum größer ist als der Grenzwert oder der Inhomogenitätsparameter größer ist als ein weiterer Grenzwert, so kann die zweite Unterdrückungsoption nach dem DIXON-Verfahren verwendet werden.

Weiterhin ist es möglich die zumindest zwei verschiedenen Unterdrückungsoptionen in Verbindung mit der Bildgebungssequenz zu speichern. Wenn die Bildgebungssequenz ausgewählt wird für den Untersuchungsablaufs, so ist damit die Kenntnis vorhanden, dass die zwei verschiedenen Unterdrückungsoptionen möglich sind und dass hier eine Auswahl getroffen werden muss.

Die automatisch ausgewählte Unterdrückungsoption kann hierbei angezeigt werden damit sie durch die Bedienperson bestätigt wird, oder die Aufnahme der MR-Signale erfolgt automatisch mit der ausgewählten Unterdrückungsoption.

Weiterhin wird eine MR-Anlage bereitgestellt, die wie oben erläutert oder nachfolgend erläutert ausgebildet ist. Die MR-Anlage weist eine Steuereinheit auf, die ausgebildet ist, das oben beschrieben oder nachfolgend beschriebene Verfahren durchzuführen.

Zusätzlich ist ein Computerprogramm vorgesehen mit Programmmitteln die direkt in eine Speichereinheit einer Steuereinheit der MR-Anlage geladen werden, um die Schritte des oben beschrieben oder nachfolgend beschrieben Verfahrens auszuführen, wenn die Programmmittel in der Steuereinheit ausgeführt werden.

Ebenso wird ein elektronisch lesbarer Datenträger bereitgestellt mit darauf gespeicherten elektronisch lesbaren Steuerinformationen. Diese sind derart ausgestaltet, dass sie bei Verwendung des Datenträgers in einer Steuereinheit der MR-Anlage das oben oder nachfolgend beschriebene Verfahren durchführen.

Die oben dargelegten Merkmale und nachfolgend beschriebenen Merkmale können nicht nur in den entsprechenden explizit dargelegten Kombinationen verwendet werden, sondern auch in weiteren Kombinationen, soweit es nicht explizit anders erwähnt ist.

Die Erfindung wird nachfolgend unter Bezugnahme auf die beiliegenden Zeichnungen näher erläutert.
Fig. 1 zeigt schematisch eine MR-Anlage, mit der erfindungsgemäß die Auswahl einer Möglichkeit zur Unterdrückung einer Gewebekomponente vereinfacht wird.
Fig. 2 zeigt schematisch die verschiedenen Optionen der Grundlage, die bei den verschiedenen Unterdrückungsoptionen arbeiten.
Fig. 3 zeigt ein Flussdiagram mit den Schritten zur automatischen Auswahl einer Unterdrückungsmöglichkeit bei einer Bildgebungssequenz.
Fig. 4 zeigt ein weiteres Flussdiagram mit weiteren Details, die bei der Auswahl einer der beiden Unterdrückungsoptionen verwendet werden können.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Weiterhin sind die Figuren schematische Darstellungen verschiedener Ausführungsformen der Erfindung. Die in den Figuren dargestellten Elemente sind nicht notwendigerweise maßstabsgetreu dargestellt. Vielmehr sind diese derart widergegeben, dass ihre Funktion und ihr Zweck für den Fachmann verständlich werden. Die in den Figuren dargestellten Verbindungen zwischen funktionellen Einheiten oder sonstigen Elementen können auch als indirekte Verbindung implementiert werden, wobei eine Verbindung drahtlos oder drahtgebunden erfolgen kann. Funktionelle Einheiten können als Hardware, Software, oder eine Kombination aus Hard- und Software implementiert werden.

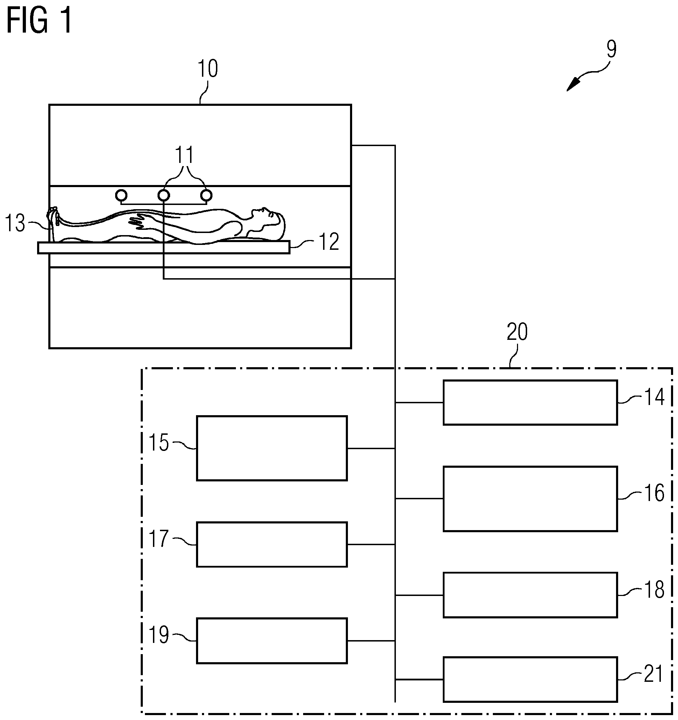

In Bezug auf Fig. 1 wird eine MR-Anlage 9 erläutert, mit der, wie nachfolgend erläutert wird, die Auswahl einer Unterdrückung einer Gewebekomponente bei der Aufnahme der MR-Signale vereinfacht wird. Die MR-Anlage 9 weist einen Magneten 10 zur Erzeugung eines Positionsfeldes B₀ auf, wobei eine auf einer Liege 12 angeordnet Untersuchungsperson 13 in den Magneten gefahren wird, um dort ortskodierte Magnetresonanzsignale aus einem bestimmten Untersuchungsabschnitt der Person 13 aufzunehmen. Zur Aufnahme der MR-Signale sind schematisch Spulen 11 dargestellt, die als Ganzkörperspulen oder Lokalspulen ausgebildet sein können. Durch die Einstrahlung von Hochfrequenzpulsen und Schalten von Magnetfeldgradienten kann, die durch das Polarisationsfeld B₀ erzeugte Magnetisierung aus der Gleichgewichtslage ausgelenkten ortskodiert werden, und die sich ergebende Magnetisierung wird von den Empfangsspulen 11 detektiert. Wie durch Einstrahlenden der HF-Pulse und durch Schalten der Magnetfeldgradienten in verschiedenen Kombinationen und Reihenfolgen MR-Bilder erzeugt werden können, ist dem Fachmann grundsätzlich bekannt und wird hier nicht näher erläutert.

Die MR-Anlage weiß weiter eine Steuereinheit 20 auf, die zur Steuerung der MR-Anlage verwendet wird. Die Steuereinheit 20 weist eine HF-Steuereinheit 14 für die Steuerung und Generierung der HF-Pulse zur Auslenkung der Magnetisierung auf. Weiterhin ist eine Gradientensteuereinheit 15 zur Steuerung und Schaltung der notwendigen Magnetfeldgradienten vorgesehen. Eine Bildsequenzsteuerung 16 steuert die Abfolge der Magnetfeldgradienten, der Signaldetektion und der HF-Pulse und damit auch die Gradientensteuereinheit 15, die Empfangsspulen und die HF-Steuereinheit 14. Über eine Eingabeeinheit 17 kann eine Bedienperson die MR-Anlage steuern, und auf einer Anzeigeeinheit 18 können MR-Bilder und sonstige zur Steuerung notwendigen Informationen angezeigt werden.

Eine Recheneinheit 19 mit mindestens einer Prozessoreinheit ist vorgesehen zur Steuerung der verschiedenen Einheiten in der Steuereinheit 20. Weiterhin ist eine Speichereinheit 21 vorgesehen, in der beispielsweise Programmmodule bzw. Programme abgespeichert werden können, die, wenn sie von der Recheneinheit 19 bzw. ihrer Prozessoreinheit ausgeführt werden, den Ablauf der MR-Anlage steuern können. Die Steuereinheit 20 bzw. die Recheneinheit 19 können wie nachfolgend erläutert ausgebildet sein, aus dem bestimmten Untersuchungsablauf zu bestimmen, ob bei einer Bildgebungssequenz eine Gewebekomponente unterdrückt werden soll. Wenn dies der Fall ist wird automatisch eine der möglichen Unterdrückungsoptionen ausgewählt.

Die nachfolgende Beschreibung erfolgt mithilfe der beiden Gewebekomponenten Fett und Wasser. Es ist jedoch möglich, dass beschriebene Verfahren bei anderen unterschiedlichen Gewebekomponenten wie Fett und Silikon, Wasser und Silikon etc. anzuwenden. Das nachfolgend beschriebene Verfahren stellt ein Verfahren vor, das der Bedienperson automatisch vorschlägt, welcher Fettunterdrückungsmodus verwendet werden soll bzw. die gewählte Unterdrückungsoption automatisch einstellt. Hierbei können Systemeigenschaften der MR-Anlage und die Lage des zu messenden Untersuchungsabschnitts in der MR-Anlage berücksichtigt werden.

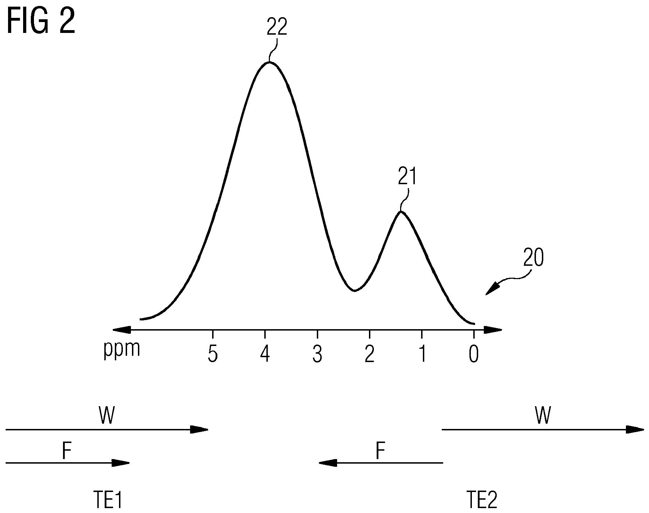

Fig. 2 zeigt schematisch ein Spektrum 20 mit den beiden Maxima 21 und 22, wobei das Maximum 21 für das Fettsignal und das Maximum 22 für das Wassersignal steht. Beide Komponenten haben ungefähr einen Frequenzunterschied von 3,5 ppm (parts per million). Wenn nun die Homogenität der MR-Komponenten der MR-Anlage 9 so gut ist, dass eine der beiden Komponenten spektral selektiv angeregt werden kann, so ist es möglich, die zu unterdrückende Gewebekomponente allein anzuregen, damit diese später bei der Aufnahme der MR-Signale kein Signal mehr liefert. Entweder erfolgt die Anregung mit der nachfolgenden Anwendung eines Gradientenpulses um die Phasenlage der angeregten Komponente zu zerstreuen, so dass kein MR-Signal mehr vorhanden ist, oder die andere weitere Möglichkeit besteht darin, die eine spektrale Komponente selektiv so anzuregen und die Signalauslese so zu wählen, dass die angeregte Komponente bei der Signalauslese keinen Signalanteil mehr hat.

Im unteren Teil ist schematisch die andere Unterdrückungsoption, die nachfolgend zweite Unterdrückungsoption genannt ist dargestellt. Durch die unterschiedliche Resonanzfrequenz haben die beiden Komponenten Fett und Wasser unterschiedliche Phasenlagen in Abhängigkeit von der Echozeit. Beispielsweise können bei einer ersten Zeit TE1 beide Komponenten die gleiche Phasenlage haben während bei einer zweiten Echozeit TE2 die beiden Komponenten eine unterschiedliche, entgegensetzte Phasenlage haben können. Durch Verwendung der Signale von zumindest einer der beiden Echozeiten ist es möglich, den Einfluss der einzelnen Komponenten einzeln zu bestimmen, wie es aus den verschiedenen DIXON-Verfahren bekannt ist.

Das Verfahren kann nun die folgenden Schritte umfassen:
In einem ersten Schritt wird das Messprotokoll gestartet, das heißt, es wird der Untersuchungsablauf bestimmt, welche Bildgebungssequenzen verwendet werden, um die MR-Bilder zu erstellen, um eine gewisse medizinische Fragestellung zu beantworten. Bei diesem Start des Untersuchungsablaufs können automatisch Schichten vorgeschlagen werden bzw. die Lage der Schichten relativ zum Untersuchungsobjekt, die Größe des Gesichtsfeldes etc. Weiterhin erfolgt üblicherweise zu Beginn eine sogenannte Shim- oder Abstimmungsprozedur, so dass bekannt ist, mit welcher Homogenität das B₀-Feld, das B₁-Feld die Messung abläuft. Zusätzlich ist damit die Lage des Untersuchungsabschnitts relativ zur MR-Anlage bekannt, nämlich ob sich der Untersuchungsabschnitt genau im Isozentrum der MR-Anlage befindet oder eher am Rand des Feldes, in dem MR-Signale aufgenommen werden können.

In einem zweiten Schritt werden für alle Protokollschritte des Untersuchungsablaufs überprüft, ob eine Sättigung einer Komponente wie beispielsweise eine Fettunterdrückung verwendet werden soll. Wenn dies der Fall ist, wird die optimale Unterdrückungsoption ermittelt und aus einer Datenbank, die beispielsweise in der Speichereinheiten 21 gespeichert ist, das passende Ablaufprotokoll für die Anwendung entnommen. Für die verschiedenen medizinischen Fragestellungen wie beispielsweise Kniemessungen oder Thoraxmessungen können in der Datenbank für jeden benötigten Kontrast die infrage kommenden Unterdrückungsoptionen hinterlegt sein. Bei der Wahl des optimalen Unterdrückungsmodus kann folgende Information berücksichtigt werden:
- es können die Systemeigenschaften und Imperfektionen der MR-Anlage berücksichtigt werden. Diese umfassen Wirbelstromtherme, B₀- und/oder B₁- Inhomogenitäten. Diese Eigenschaften unterscheiden sich von MR-Anlage zu MR-Anlage und hängen von vielen Faktoren wie der Geometrie des Untersuchungsabschnitts ab. Diese Eigenschaften und Inhomogenitäten sind jedoch bei Start der eigentlichen MR-Messung üblicherweise bekannt, da diese zu Beginn vor dem Start der eigentlichen MR-Messung in einem Abstimmungsmodus bestimmt und optimiert werden falls notwendig.
- Zusätzlich kann die Lage des zu messenden Untersuchungsabschnitts bezüglich des Isozentrums und die benötigte Abdeckung, das heißt die Größe des Gesichtsfeldes bestimmt werden. Diese Information kann entweder von der Bedienperson vorgegeben sein oder kann bei automatischen Abläufen aus dem obigen Schritt entnommen werden.

Aus der Kombination dieser beiden Faktoren wird nun bestimmt, ob der Untersuchungsabschnitt noch in einem Bereich liegt, in dem eine spektrale Sättigung mittels eines HF-Pulses möglich ist. Da die oben genannten Inhomogenitäten im Allgemeinen mit steigendem Abstand zum Isozentrum zu nehmen, kann beispielsweise ab einem bestimmten Abstand zum Isozentrum automatisch auf die Unterdrückungsoption gewechselt werden, die die unterschiedliche Phasenlage nach DIXON verwendet. In den Außenbereichen der MR-Anlage liegen die Frequenzfehler durch Wirbelströme oder Feldinhomogenitäten in einem Bereich liegen, der dem spektralen Frequenzunterschied zwischen Fett und Wasser entspricht. Der Abstand zum Isozentrum kann hierbei als relative Größe, z.B. als Prozentsatz zu dem maximal möglichen Gesichtsfeld bestimmt werden. Liegt z.B. der Messbereich in einem Abschnitt, der unterhalb von 50, 60 oder 70 % des maximalen Gesichtsfeldes liegt, so wird die spektrale Sättigung gewählt und außerhalb die DIXON-Methode.

Am Beispiel einer Knieuntersuchung würde dies bedeuten, dass anhand der Lage und der Ausdehnung des Knies und den in diesem Bereich vorliegen Wirbelströmen, den B₀-Inhomogenitäten oder B₁-Inhomogenitäten bestimmt wird, welche Unterdrückungsoption und damit welche Bildgebungssequenzen verwendet wird.

In einem dritten Schritt wird der nun so bestimmte Ablauf entweder der Bedienperson vorgeschlagen oder automatisch in den Untersuchungsablauf integriert, so dass das Verfahren ohne jegliche Interaktion mit der Bedienperson ablaufen kann.

Weiterhin kann das Verfahren auch so ausgestaltet sein, dass je nach dem oben genannten Parametern wie der Abstand zum Zentrum und den Inhomogenitäten nur bestimmte Unterdrückungsoptionen zugelassen bzw. anwählbar sind. Beispielsweise kann in Bereichen mit bekannten sehr hohen Wirbelströmen die eine Unterdrückungsoption mit der spektralen Sättigung nicht zugelassen werden. Hier sieht die Bedienperson bereits aus der Darstellung des Untersuchungsablaufs, dass im vorliegenden Beispiel keine spektrale Fettsättigung möglich sein kann.

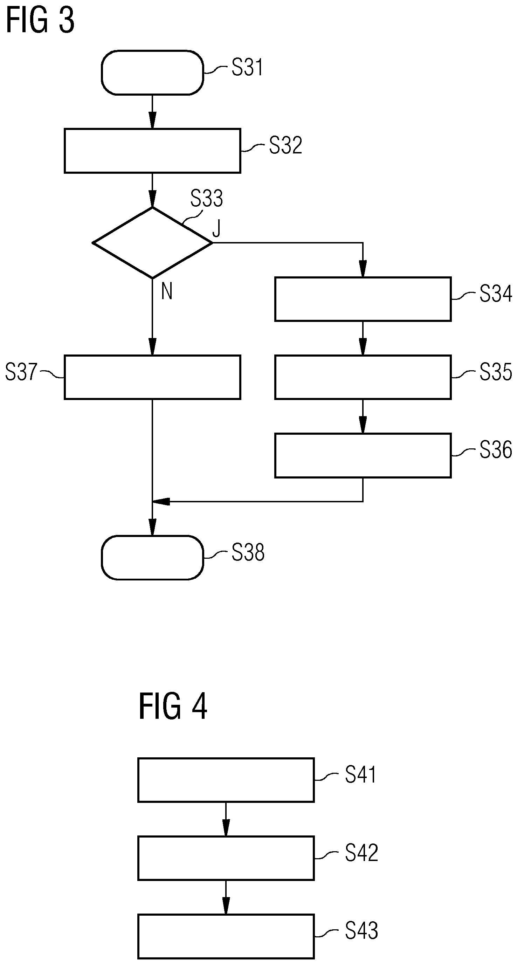

Fig. 3 fast schematisch einige Schritte des Verfahrens zusammen. Das Verfahren startet in einem Schritt S31 und in einem Schritt S32 wird der Untersuchungsablauf für die Untersuchungsperson bestimmt, wobei hierbei beispielsweise festgelegt wird, dass Aufnahmen eines bestimmten Körperteils wie Knie, Schulter, oder Kopf notwendig sind. In einem Schritt S33 wird untersucht, ob bei dem Untersuchungsablauf eine Bildgebungssequenz verwendet wird, für die unterschiedliche Unterdrückungsoptionen für die Unterdrückung einer Gewebekomponente möglich sind und eine Unterdrückung gewünscht ist. Dies ist möglich, da beispielsweise für jede Bildgebungssequenz im Speicher hinterlegt ist, welche theoretisch möglichen Unterdrückungsoptionen wählbar sind. Wird in Schritt S33 festgestellt, dass eine derartige Bildgebungssequenz verwendet werden soll, so wird in Schritt S34 der Ablaufparameter der Untersuchung bestimmt. In den oben genannten Beispielen enthielt der Ablaufparameter einen Parameter über die Inhomogenität und/oder einen Parameter über den Abstand vom Isozentrum. Wenn einer oder mehrerer dieser Parameter bestimmt werden, so kann in Abhängigkeit von einem oder mehreren dieser Parameter festgelegt werden, welche Unterdrückungsoption ausgewählt wird in Schritt S35. Schließlich wird in Schritt 36 die Bildgebungssequenz mit der entsprechenden Unterdrückungsoption aufgenommen. Falls im Schritt S33 festgestellt wird, dass keine Optionen bestehen bei der Wahl der Unterdrückungsoptionen erfolgt in Schritt S37 die Auswahl der Bildgebungssequenz gemäß des ausgewählten Untersuchungsablaufs ohne Option auf Unterdrückung einer Gewebekomponente. Das Verfahren endet in Schritt S38.

In Fig. 4 ist die Bestimmung des Ablaufparameters von Schritt S34 näher erläutert. In Schritt S41 kann hierbei der Parameter bestimmt werden, der ein Inhomogenitätsparameter ist und die Inhomogenitäten der Komponenten der MR-Anlage festlegt, wie beispielsweise die Inhomogenität des B₀-Feldes oder die zu erwartenden Wirbelströme. In einem Schritt S42 wird weiterhin überprüft, in welchem Abstand zum Isozentrum die Aufnahme der MR-Signale erfolgen soll. Dies kann aus der ausgewählten Schichtposition bestimmt werden. Diese beiden Parameter zusammen können nun verwendet werden, um in einem Schritt S43 schließlich eine der beiden Unterdrückungsoptionen auszuwählen wie oben erläutert. Ist der Abstand kleiner als ein Grenzwert und ist die Inhomogenität kleiner als ein Grenzwert, kann die erste Option gewählt werden. Ansonsten wird die zweite Option gewählt.

Zusammenfassend ermöglicht die oben beschriebene Erfindung eine automatische Wahl einer vorteilhaften Optionsunterdrückung einer Gewebekomponente, wobei Systemeigenschaften wie Lagerung und die Physiologie der Untersuchungsperson berücksichtigt werden.

Das Verfahren bietet vor allem bei unerfahrenen Anwendern den Vorteil, dass ihm die Wahl und das nötige Verständnis für die Auswahl der passenden Bildgebungssequenz abgenommen werden, ohne dass er darauf Einfluss nehmen muss. Dadurch können Fehler in Artefakte in den Bildern, die beispielsweise eine erneute Untersuchung nötig machen würden, vermieden werden.

## Patentansprüche

1. Verfahren zum automatischen Steuern eines Untersuchungsablaufs in einer MR-Anlage bei der Aufnahme von MR-Signalen in einem Untersuchungsabschnitt einer Untersuchungsperson (13), der zwei Gewebekomponenten mit zwei unterschiedlichen MR-Resonanzfrequenzen aufweist, mit den Schritten:
- Bestimmen eines Untersuchungsablaufs für die Untersuchung des Untersuchungsabschnitts,
- Bestimmen, ob der Untersuchungsablauf eine Bildgebungssequenz aufweist, bei der eine der beiden Gewebekomponenten unterdrückt werden soll und für die zumindest zwei verschiedene Unterdrückungsoptionen existieren, diese eine Gewebekomponente bei der MR-Signalaufnahme zu reduzieren, wobei wenn dies der Fall ist:
- Bestimmen eines Ablaufparameters der Untersuchung,
- Auswählen eine der beiden Unterdrückungsoptionen in Abhängigkeit von dem bestimmten Ablaufparameter für die Bildgebungssequenz.

2. Verfahren nach Anspruch 1, wobei das Bestimmen des Ablaufparameters beinhaltet, zumindest einen Inhomogenitätsparameter der MR-Anlage zu bestimmen, wobei die eine der beiden Unterdrückungsoptionen automatisch ausgewählt wird unter Berücksichtigung des bestimmten Inhomogenitätsparameter.

3. Verfahren nach Anspruch 2, wobei der Inhomogenitätsparameter zumindest einen der folgenden Parameter aufweist: eine Homogenität des Polarisationsfeldes Bo, eine Homogenität des B₁ Feldes zur Einstrahlung von HF-Pulsen in den Untersuchungsabschnitt, eine Größe eines induzierten Wirbelstroms.

4. Verfahren nach einem der vorstehenden Ansprüche, wobei das Bestimmen des Ablaufparameters aufweist, in welchem Abstand zu einem Zentrum der MR-Ablage der Untersuchungsabschnitt bei der Aufnahme der MR-Signale positioniert ist, wobei die eine der beiden Unterdrückungsoption automatisch ausgewählt wird unter Berücksichtigung des bestimmten Abstands.

5. Verfahren nach einem der vorstehenden Ansprüche, wobei die zumindest zwei verschiedenen Unterdrückungsoptionen eine erste Unterdrückungsoption, bei der die beiden Gewebekomponenten mit einem spektral selektiven HF-Puls unterschiedlich angeregt werden, und eine zweite Unterdrückungsoption aufweisen, bei der eine unterschiedliche Phasenlage der beiden Gewebekomponenten zu einer Echozeit verwendet wird.

6. Verfahren nach Anspruch 4 und 5, wobei die zweite Unterdrückungsoption gewählt wird, wenn der Abstand zum Zentrum größer als ein erster Grenzwert ist.

7. Verfahren nach Anspruch 2 und 5, wobei die zweite Unterdrückungsoption gewählt wird, wenn der Inhomogenitätsparameter größer als ein zweiter Grenzwert ist.

8. Verfahren nach Anspruch 2, 4 und 5, wobei die erste Unterdrückungsoption gewählt wird, wenn der Inhomogenitätsparameter kleiner als ein zweiter Grenzwert und der Abstand vom Zentrum kleiner als ein erster Grenzwert ist.

9. Verfahren nach Anspruch 2, 4 und 5, wobei die zweite Unterdrückungsoption gewählt wird, wenn entweder der Abstand zum Zentrum größer als ein erster Grenzwert ist oder wenn der Inhomogenitätsparameter größer als ein zweiter Grenzwert ist.

10. Verfahren nach einem der vorstehenden Ansprüche, wobei die zumindest zwei verschiedenen Unterdrückungsoptionen verbunden mit der Bildgebungssequenz gespeichert sind.

11. Verfahren nach einem der vorstehenden Ansprüche, wobei die ausgewählte Unterdrückungsoption angezeigt wird für eine Bestätigung durch eine Bedienperson, welche den Untersuchungsablauf steuert, oder die Aufnahme der MR-Signale aus dem Untersuchungsabschnitt erfolgt automatisch mit der Bildgebungssequenz und der ausgewählten Unterdrückungsoption.

12. MR-Anlage (9), welche ausgebildet ist zum automatischen Steuern eines Untersuchungsablaufs bei der Aufnahme von MR-Signalen in einem Untersuchungsabschnitt einer Untersuchungsperson, der zwei Gewebekomponenten mit zwei unterschiedlichen MR-Resonanzfrequenzen aufweist, wobei die MR-Anlage einer Steuereinheit (20) aufweist, die ausgebildet ist, ein Verfahren nach einem der Ansprüche 1 bis 11 durchzuführen.

13. Computerprogramm, welches Programmmittel umfasst und direkt in eine Speichereinheit einer Steuereinheit einer MR-Anlage ladbar ist, um alle Schritte des Verfahrens nach einem der Ansprüche 1-11 auszuführen, wenn die Programmmittel in der Steuereinheit ausgeführt werden.

14. Elektronisch lesbarer Datenträger mit darauf gespeicherten elektronisch lesbaren Steuerinformationen, welche derart ausgestaltet sind, dass sie bei Verwendung des Datenträgers in einer Steuereinheit einer MR-Anlage das Verfahren nach einem der Ansprüche 1-11 durchführen.
